# EUROPEAN PATENT APPLICATION

(11) **EP 4 696 359 A1**
(43) Date of publication of application: **18.02.2026**
(21) Application number: 23934961.6
(22) Date of filing: 30.10.2023
(51) Int. Cl.: A61M 25/10, A61M 25/00

(54) **BALLOON CATHETER**

(30) Priority: 23.04.2023 CN 202310444195
(71) Applicant: Shanghai MicroPort Medical (Group) Co., Ltd., Shanghai 201203 (CN)
(72) Inventor: HU, Meng, Shanghai 201203 (CN); ZHOU, Qi, Shanghai 201203 (CN); CAO, Chunhong, Shanghai 201203 (CN); WANG, Jing, Shanghai 201203 (CN); ZHANG, Zhenghai, Shanghai 201203 (CN)
(74) Representative: Marks & Clerk LLP
(86) International application number: PCT/CN2023/127544
(87) International publication number: WO 2024/221793

(57) **Abstract**

A balloon catheter includes a balloon body (100) and a covering membrane (200). The balloon body (100) defines an inflation lumen, through which an inflation medium can be introduced into the balloon body (100) to inflate it. When so inflated, the balloon body (100) has a cross-section with raised portions (110) and recessed portions (120), which alternate circumferentially around the balloon body (100). The covering membrane (200) is disposed on a circumferential surface of the balloon body (100) and attached to the raised portions (110) so that fluid flow channels (101) are defined between the covering membrane (200) and the recessed portions (120), which are isolated from the inflation lumen. The balloon body (100) of the balloon catheter can be introduced into a blood vessel to dilate it without blocking blood flow therein. Thus, the balloon body (100) can be deployed in an expanded configuration for an extended period of time, resulting in improved therapeutic outcomes.

## Description

### TECHNICAL FIELD

The present invention pertains to the field of medical devices, and more particularly relates to a balloon catheter.

### BACKGROUND

Balloon angioplasty was originally used to treat peripheral arterial disease, and percutaneous transluminal coronary angioplasty (PTCA) that emerged at a later time has offered a new option for the treatment of coronary artery disease. Bare-metal stents, despite their success in addressing to some extent potential complications of balloon dilation, including vessel wall dissection and acute vascular occlusion, are prone to the development of in-stent restenosis (ISR). The demand for reducing the incidence of ISR has given rise to drug-eluting stents (DESs). When deployed in a diseased blood vessel, a DES can continually release an immunosuppressive or anti-tumor drug over a prolonged period of time, effectively excessive neointimal growth of the blood vessel. However, this inhibition of intimal hyperplasia may lead to delayed endothelial healing. Further, DES implantation requires prolonged dual antiplatelet therapy, significantly increasing the risk of post-implantation bleeding.

A drug-coated balloon (DCB) can be obtained by applying a drug coating to the surface of a balloon. After a DCB is delivered into a blood vessel, it can be inflated into contact with the wall of the blood vessel. Consequently, the drug coating will separate from the balloon surface and instead adhere to the blood vessel wall, thus delivering its intended therapeutic effect on a lesion site in the blood vessel. DCBs offer many advantages as follows:
1) A drug can be brought into direct contact with a vessel wall and thereby uniformly delivered to a damaged area of the wall.
2) At an early stage of treatment with the most pronounced progression of neointimal growth, a high drug concentration can be obtained on a vessel wall, enabling effective inhibition of intimal hyperplasia. At a late stage of treatment, a much lower drug concentration can be obtained on a vessel wall, which is in favor of endothelialization and associated with a reduced risk of thrombosis.
3) Drug release can be accomplished without reliance on a polymer matrix, reducing the risk of inflammation.
4) The associated procedures are simpler and require shorter radiation exposure and reduced use of contrast media, providing increased surgical safety.
5) They exhibit excellent crossability through small vessel lesions, tortuous lesions and bifurcation lesions and do not require the use of a metal scaffold. After being treated with a DCB, a blood vessel can largely restore the original anatomy, with its blood flow pattern being less affected. Compared with double-layer stents, the problem of a reduced vascular lumen is avoided during ISR treatment.
6) Implantation of foreign matter can be avoided, leaving the patient with a chance of receiving subsequent treatment if necessary.
7) They allow for shorter dual antiplatelet medication, reducing the incidence of bleeding complications and associated medical cost. Therefore, they are particularly suitable for patients who are intolerant to antiplatelet therapy or have recently received surgical treatment.

However, existing DCBs are still associated with some inherent drawbacks. For example, a DCB, when deployed in an expanded configuration in a blood vessel, will block blood flow in the vessel and therefore has to be deployed in this configured only for a limited period of time. This is because severe ischemia of distal tissues may be otherwise caused, which is detrimental to the patient's well-being. On the other hand, such short-term balloon dilation may disallow adequate absorption of the drug coating by the vessel wall. As a consequence, transfer of the drug may be too inefficient to enable satisfactory eventual therapeutic outcomes.

### SUMMARY

It is an object of the present invention to provide a balloon catheter, which can be expanded in a blood vessel without occluding it.

To this end, the present invention provides a balloon catheter including a balloon body and a covering membrane. The balloon body defines an inflation lumen configured to allow an inflation medium to be introduced therein to inflate the balloon body. When so inflated, the balloon body has a cross-section with raised and recessed portions alternating circumferentially around the balloon body. The covering membrane is disposed on a circumferential surface of the balloon body and attached to the raised portions, fluid flow channels are defined between the covering membrane and the recessed portions, the fluid flow channels are isolated from the inflation lumen.

Optionally, there may be a plurality of raised portions, which are evenly arranged circumferentially around the balloon body in the cross-section thereof.

Optionally, the number of raised portions may be equal to the number of recessed portions.

Optionally, the number of raised portions may range from two to six.

Optionally, the balloon catheter may further include supports provided in at least some of the recessed portions, which extend along an axis of the balloon body and are attached to both the recessed portions and the covering membrane.

Optionally, each support may have a dimension measured along a radial direction of the balloon body, which is equal to the difference between a radius of a circle defined by apices of all the raised portions and a radius of a circle defined by bottoms of all the recessed portions.

Optionally, each support may have a cross-section in the shape of a circular annulus with an outer diameter, which is equal to the difference between the radius of the circle defined by the apices of all the raised portions and the radius of the circle defined by the bottoms of all the recessed portions.

Optionally, the circle defined by the apices of all the raised portions may have a diameter in the range of 2 mm to 5 mm, and the circle defined by the bottoms of all the recessed portions may have a diameter in the range of 1 mm to 2.5 mm.

Optionally, the covering membrane may be provided with a drug coating on an outer surface thereof.

Optionally, the balloon catheter may further include a catheter assembly defining an inflation medium channel, wherein the balloon body is disposed over an outer circumferential surface of the catheter assembly around a distal end thereof, and the inflation lumen of the balloon body communicates with the inflation medium channel.

Optionally, the catheter assembly may include an outer tube and an inner tube, the inner tube partially inserted within the outer tube, with its distal end being located external to a distal end of the outer tube, wherein the inflation medium channel is defined between the inner and outer tubes; the balloon body is disposed over an outer circumferential surface of the inner tube around a distal end thereof; and a proximal end of the balloon body is coupled to the distal end of the outer tube.

The present invention offers the following advantages over the prior art:
It provides a balloon catheter including a balloon body and a covering membrane. The balloon body defines an inflation lumen configured to allow an inflation medium to be introduced therein to inflate the balloon body. When so inflated, the balloon body has a cross-section with raised and recessed portions alternating circumferentially around the balloon body. The covering membrane is disposed on a circumferential surface of the balloon body and attached to the raised portions so that fluid flow channels are defined between the covering membrane and the recessed portions, which are isolated from the inflation lumen. After the balloon body and the covering membrane in the balloon catheter are delivered into a blood vessel in a patient, the inflation medium may be introduced into the inflation lumen to inflate the balloon body. Supported by the balloon body, an outer surface of the covering membrane at the portions where it is attached to the raised portions can be brought into contact with a wall of the blood vessel. In this way, the blood vessel wall can be supported and stretched, dilating the blood vessel. Moreover, when a drug coating is present on the outer surface of the covering membrane, a drug contained in the drug coating can be adsorbed by the blood vessel wall. At the same time, due to the presence of the fluid flow channels, blood flow is still allowed through the fluid flow channels toward downstream tissues. That is, when the balloon catheter is used in an expanded configuration in a blood vessel, the expansion of the balloon body will not lead to occlusion of the blood vessel, and hence will not cause ischemia of downstream tissues. Therefore, the balloon body is allowed to be deployed in the expanded configuration for an extended period of time, thus providing better dilation of the blood vessel and resulting in more efficient drug absorption of the blood vessel wall and hence improved therapeutic outcomes.

Further, the balloon catheter may include supports provided in at least some of the recessed portions, which extend along an axis of the balloon body and are attached to both the recessed portions and the covering membrane. Additionally, each support may have a dimension measured along a radial direction of the balloon body, which is equal to the difference between a radius of a circle defined by apices of all the raised portions and a radius of a circle defined by bottoms of all the recessed portions. With additional support from the supports, a cross-sectional shape of the covering membrane can be made closer to a circle, allowing it to provide better support to a blood vessel wall. Moreover, a drug coating on the covering membrane can be brought into contact with the blood vessel wall over a larger area. Further, the balloon body can be more easily fabricated.

### BRIEF DESCRIPTION OF THE DRAWINGS

The accompanying drawings are provided to facilitate a better understanding of the present invention and do not unduly limit the scope thereof in any sense, in which:
Fig. 1 schematically illustrates a distal portion of a balloon catheter according to an embodiment of the present invention;
Fig. 2 schematically illustrates a cross-section of the distal portion of the balloon catheter of Fig. 1, which has three raised portions and three recessed portions;
Fig. 3 schematically illustrates a distal portion of a balloon catheter according to another embodiment of the present invention, a cross-section of which has four raised portions and four recessed portions;
Fig. 4 is a schematic cross-sectional view of the distal portion of the balloon catheter of Fig. 3;
Fig. 5 schematically illustrates a balloon body of the balloon catheter of Fig. 3;
Fig. 6 schematically illustrates a proximal portion of a balloon catheter according to an embodiment of the present invention;
Fig. 7 schematically illustrates an application scenario of a balloon catheter according to an embodiment of the present invention; and
Fig. 8 schematically illustrates a crimped and folded balloon body of the balloon catheter of Fig. 3.

### List of Reference Numerals

100 balloon body; 110 raised portion; 120 recessed portion; 121 second circle; 101 fluid flow channel; 200 covering membrane; 300 drug coating; 400 catheter assembly; 410 outer tube; 411 radiopaque band; 420 inner tube; 500 support; 600 guide tip; 700 radiopaque element; 800 connector; 900 stress diffusion tube.

### DETAILED DESCRIPTION

Embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "a plurality of" means "two or more", unless the context clearly dictates otherwise. As used herein, the term "or" is generally employed in the sense including "and/or", unless the context clearly dictates otherwise. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

As used herein, the terms "proximal" and "distal" may be used to describe relative positions and locations of various components and elements of a medical device. While not intended to be limiting, a "distal end" usually refers to an end that enters the patient's body first, while a "proximal end" usually refers to an end closer to an operator, during normal operation of the medical device.

It is an object of the present invention to provide a balloon catheter having a balloon body around its distal end, which can be expanded in a blood vessel without occluding the blood vessel or blocking blood flow therein, preventing ischemia of downstream tissues that may otherwise occur. Therefore, the balloon body is allowed to be deployed in an expanded configuration for an extended period of time, resulting in improved therapeutic outcomes.

Objects, advantages and features of the present invention will become more apparent from the following detailed description of examples of embodiment thereof. Note that the figures are provided in a very simplified form not necessarily drawn to exact scale for the only purpose of helping to explain the disclosed embodiments in a more convenient and clearer way. Throughout these drawings, like numerals indicate like elements.

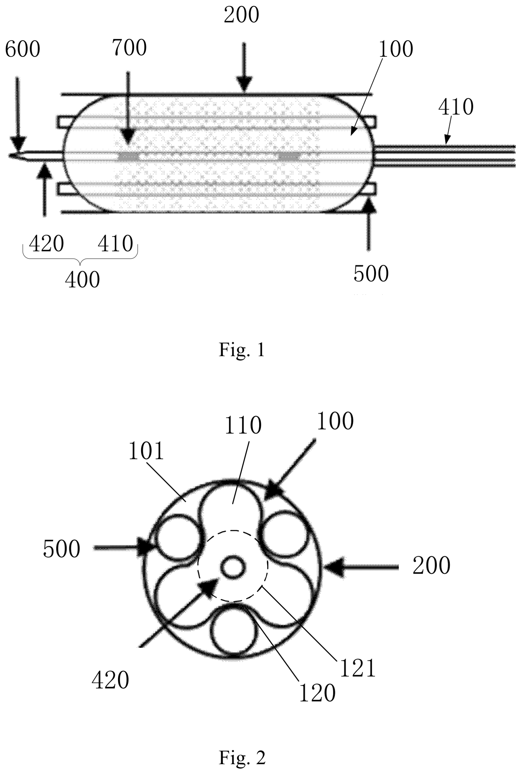

As shown in Figs. 1 to 5, embodiments of the present invention provide a balloon catheter including a balloon body 100 and a covering membrane 200. The balloon body 100 defines an inflation lumen, and an inflation medium can be introduced into the inflation lumen to inflate the balloon body 100 to a larger radial dimension. When the inflation medium is discharged from the inflation lumen, the balloon body 100 will contract to a smaller radial dimension. When in an inflated configuration, the balloon body 100 defines a cross-section with raised portions 110 and recessed portions 120, which alternate circumferentially around the balloon body 100. The covering membrane 200 is disposed over a circumferential surface of the balloon body 100 and attached to the raised portions 110, thereby defining fluid flow channels 101 between the covering membrane 200 and the recessed portions 120, the fluid flow channels 101 are isolated from the inflation lumen.

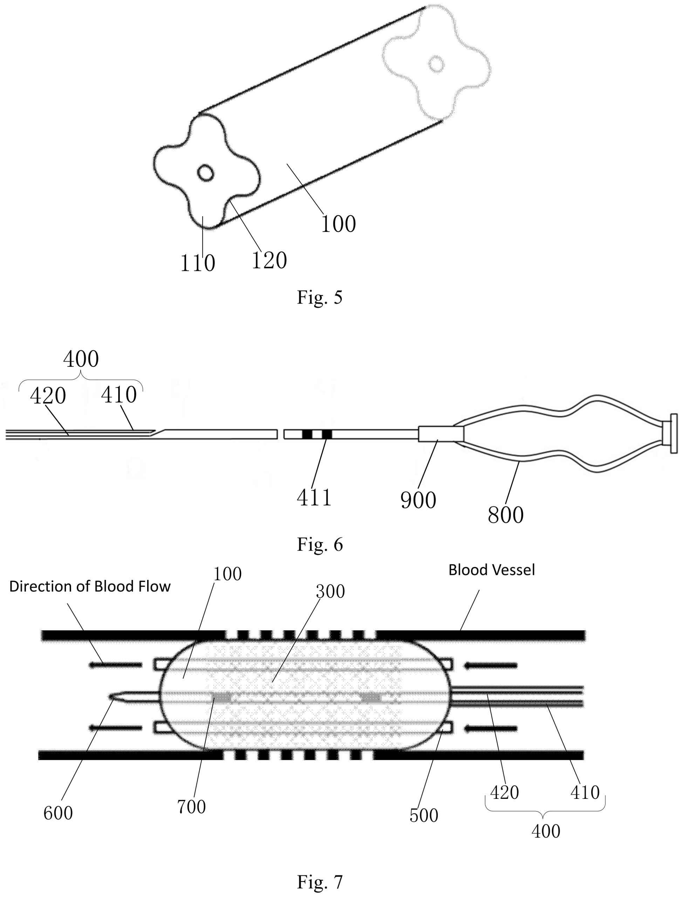

It is known to those skilled in the art that the balloon catheter further includes a catheter assembly 400 (as shown in Fig. 6) defining an inflation medium channel (not shown) configured for flow of the inflation medium therethrough. The balloon body 100 is disposed over an outer circumferential surface of the catheter assembly 400 around a distal end thereof, and the inflation lumen of the balloon body 100 communicates with the inflation medium channel, allowing the inflation medium to flow into the inflation lumen of the balloon body 100 via the inflation medium channel, or to be discharged from the inflation lumen and flow out through the inflation medium channel.

As shown in Fig. 7, during use of the balloon catheter, the balloon body 100 may be crimped (to contract to a smaller radial dimension) and then advanced into a blood vessel. When the balloon body 100 reaches a predetermined position, the inflation medium may be introduced into the inflation lumen of the balloon body 100, thereby inflating the balloon body 100. As a result, the covering membrane 200 is expanded and stretched by the balloon body 100, and the outer surface of the covering membrane 200 at the portions where it is attached to the balloon body 100 is brought into contact with the wall of the blood vessel, supporting and dilating the blood vessel. Preferably, a drug coating 300 is further provided on the outer surface of the covering membrane 200. In this case, the drug coating 300 over the outer surface of the covering membrane 200 at the portions where it is attached to the balloon body 100 can be brought into contact with the blood vessel wall, allowing a drug contained in the drug coating 300 to be absorbed by the blood vessel wall. At the same time, due to the presence of the fluid flow channels 101, blood flow is still allowed in the blood vessel through the fluid flow channels 101 (as shown in Fig. 7) toward downstream tissues, without being blocked, thus avoiding ischemia of the downstream tissues that may otherwise occur. In other words, since the balloon catheter can be used in a blood vessel without occluding it, the balloon body 100 is allowed to be deployed in an expanded configuration for an extended period of time, thus providing better dilation of the blood vessel and maintaining its wall in contact with the drug coating 300 on the outer surface of the covering membrane 200 for a longer time. As a result, the drug can be more efficiently transferred and adsorbed, resulting in improved therapeutic outcomes.

According to embodiments of the present invention, the balloon body 100 may be made of any of polyamide, polyether block amide and polyurethane. In practical applications, the balloon body 100 may have an axial length of 10 mm to 40 mm. The covering membrane 200 may be any elastic polymer film. The covering membrane 200 may be attached to the raised portions 110 of the balloon body 100 by adhesive bonding, welding or any other suitable method. The covering membrane 200 may have an axial length equal to that of the balloon body 100. The drug coating 300 contains at least an active ingredient. The drug coating 300 may further contain a carrier matrix. The active ingredient may be an anti-proliferative drug, which can reduce the risk of vascular restenosis. Examples of the active ingredient may include, but are not limited to, at least one of rapamycin, rapamycin derivatives, paclitaxel and paclitaxel derivatives. Examples of the carrier matrix may include, but are not limited to, at least one of iopromide, urea, lecithin and polylactic acid. The drug coating 300 may be formed on the outer surface of the covering membrane 200 by electrostatic spraying, atomized spraying, dip coating or crystal growth.

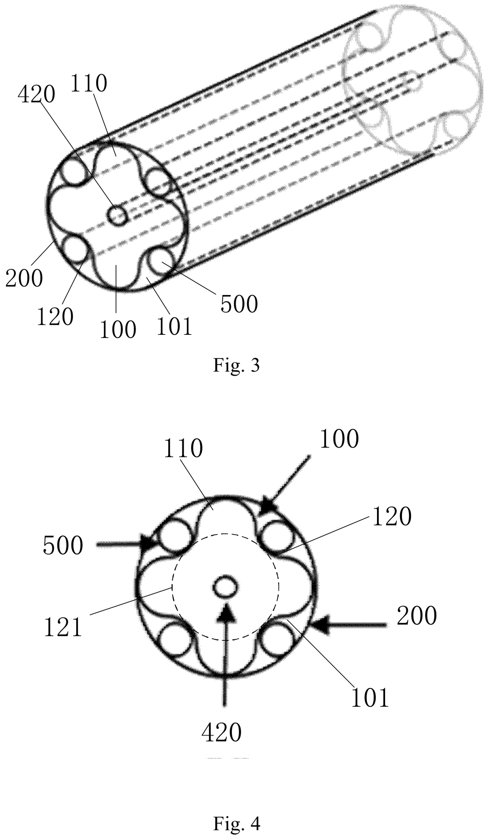

Typically, there are a plurality of raised portions 110, the multiple said raised portions 110 are uniformly arranged circumferentially around the cross-section of the balloon body 100. With this arrangement, when in the inflated configuration, the balloon body 100 can provide circumferentially uniform support to the covering membrane 200 and hence to the blood vessel wall. Usually, the number of recessed portions 120 is equal to that of raised portions 110. Optionally, the number of raised portions 110 is 2-6; thus, the number of the recessed portions 120 is also 2-6. For example, in some embodiments, as shown in Figs. 1 and 2, there are three raised portions 110 and three recessed portions 120. In alternative embodiments, as shown in Figs. 3 to 5, there are four raised portions 110 and four recessed portions 120.

Apices of all the raised portions 110 are located on a single circle, referred to hereinafter as the first circle. For each raised portion 110, the apex is defined as the point thereon that is spaced from an axis of the balloon body 100 at a maximum distance. The first circle has a diameter, which may depend on an inner diameter of a target blood vessel in need of treatment. Typically, the diameter is in the range of 2 mm to 5 mm, such as 4 mm. Bottoms of all the recessed portions 120 are located on another single circle, referred to hereinafter as the second circle 121. For each recessed portion 120, the bottom is defined as the point thereon that is spaced from the axis of the balloon body 100 at a minimum distance. If a diameter of the second circle 121 is too small, the balloon body 100 would be difficult to fabricate. On the contrary, if the diameter is too large, a flux area of the fluid flow channels 101 may be too small to impart desirable hemodynamic properties to blood flow in a blood vessel. In practice, the diameter of the second circle 121 is selected from the range from 1 mm to 2.5 mm, such as 2 mm.

In general terms, when the balloon body 100 is deployed in the inflated configuration in a blood vessel, a greater number of raised portions 110 will lead to a cross-section of the covering membrane 200 closer to the first circle, stronger support provided by the balloon body 100 to the wall of the blood vessel and better dilation of the blood vessel. On the other hand, this will also lead to higher fabrication complexity of the balloon body 100 and a reduced flux area of the fluid flow channels 101, which is unfavorable to blood flow in the vessel.

In view of the above, the balloon catheter further includes supports 500, the supports 500 are provided in at least some of the recessed portions 120, and the supports 500 extend along the axis of the balloon body 100. The supports 500 are attached to both the recessed portions 120 and the covering membrane 200. This can avoid dislodgement of the supports 500. Each support 500 may have a dimension measured along a radial direction of the balloon body 100, which is equal to the difference between a radius of the first circle and a radius of the second circle 121. With this arrangement, the supports 500 in the recessed portions 120 can provide radial support to the covering membrane 200 when the balloon body 100 is in the inflated configuration, making the cross-section of the covering membrane 200 as close as possible to the first circle. Moreover, the balloon catheter can support a blood vessel wall at more points, providing an improved vascular dilation effect. The supports 500 may be attached to the balloon body 100 and the covering membrane 200 by gluing or welding.

The presence of the supports 500 would definitely reduce the flux area of the fluid flow channels 101, which is not conducive to blood flow. To mitigate this, the supports 500 are preferred to be hollow, that is, each support 500 is a tubular structure. This way, blood can also flow through the inner lumen of each support 500. As would be appreciated, a smaller wall thickness of the supports 500 will lead to a less adverse impact that they have on the flux area of the fluid flow channels 101. In a preferred embodiment, each support 500 has a cross-section in the shape of a circular annulus having an outer diameter, which is equal to the difference between the radius of the first circle and the radius of the second circle 121, and an inner diameter optionally of 1 mm.

The supports 500 may be made of a metal material, or a polymer material. Examples of the metal material may include, but are not limited to, at least one of medical stainless steel and medical cobalt-based alloys. Examples of the polymer material may include, but are not limited to, at least one of polyethylene terephthalate and polyether block amide.

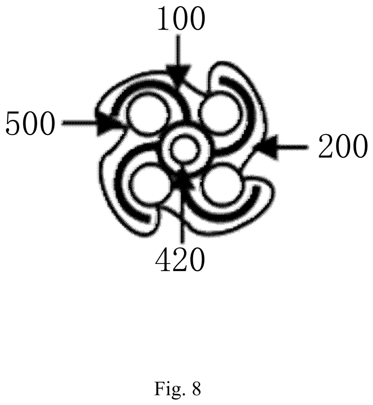

According to embodiments of the present invention, the balloon body 100 may be crimped so that each raised portion 110 is folded into a curved flap, as shown in Fig. 8. Moreover, in this crimped configuration, there are as many curved flaps as raised portions 110, and the supports 500 remain in the recessed portions 120 and are wrapped by the folded raised portions 110. This is advantageous in a reduced space occupied by the supports 500 and a reduced outer diameter of the crimped balloon catheter, which allows a distal end of the balloon catheter to be more easily inserted into and advanced within a blood vessel.

According to embodiments of the present invention, referring again to Fig. 6, the catheter assembly 400 includes an outer tube 410 and an inner tube 420. The inner tube 420 is partially inserted inside the outer tube 410, the distal end of the inner tube 420 is located external to a distal end of the outer tube 410. The inflation medium channel is defined between an outer surface of the inner tube 420 and an inner surface of the outer tube 410, and the distal end of the outer tube 410 is also sealingly attached to the inner tube 420. The balloon body 100 is disposed over an outer circumferential surface of the distal end of the inner tube 420, and a proximal end of the balloon body 100 is also attached to the distal end of the outer tube 410 so that the inflation lumen of the balloon body 100 is brought into communication with the inflation medium channel.

With continued reference to Fig. 6, as is conventional, the proximal end of the outer tube 410 is provided with a first inlet (not labeled), and the proximal end of the inner tube 420 is provided with a second inlet (not labeled). The first and second inlets and a lumen of the inner tube 420 together define a guide-wire passage configured for passage of a guide wire therethrough. Additionally, the proximal end of the outer tube 410 is provided on its outer surface with a radiopaque band 411, the radiopaque band 411 is configured to help determine a length of the balloon catheter that has been inserted into the body of a patient during use.

As shown in Figs. 1 and 7, the balloon catheter further includes a guide tip 600. The guide tip 600 is provided at the distal end of the inner tube 420 on a distal side of the balloon body 100. It is typically a tapered structure with an outer diameter gradually decreasing from proximal to distal, and is provided to guide the balloon body 100 into a patient's body.

The balloon catheter further includes a radiopaque element 700. The radiopaque element 700 is provided on the outer surface of the inner tube 420 around the distal end thereof, which is configured to indicate the position of the balloon body 100. This radiopaque element 700 may be visible in X-ray images, allowing an operator to visually identify the position of the balloon body 100 in a blood vessel.

As shown in Fig. 6, the balloon catheter further includes a connector 800. The connector 800 is connected to a proximal end of the catheter assembly 400 via a stress diffusion tube 900. The connector 800 is brought into communication with the inflation medium channel and connected to an external irrigation and aspiration mechanism, thereby allowing the inflation agent to be perfused into the inflation lumen of the balloon body 100 or allowing the inflation agent to be discharged from the inflation lumen.

As discussed above, according to embodiments of the present invention, the balloon body can be used to dilate a blood vessel without blocking blood flow therein, avoiding downstream ischemia. Therefore, the balloon body is allowed to be deployed in an inflated configuration for an extended period of time, thus providing better dilation of the blood vessel and maintaining its wall in contact with the drug coating on the outer surface of the covering membrane for a longer time. As a result, the drug can be more efficiently transferred to and adsorbed by the blood vessel wall, resulting in improved therapeutic outcomes.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A balloon catheter, comprising a balloon body and a covering membrane, the balloon body defining an inflation lumen, the inflation lumen configured to allow an inflation medium to be introduced therein to inflate the balloon body, the balloon body in an inflated state having a cross-section with raised portions and recessed portions alternating circumferentially around the balloon body, the covering membrane disposed on a circumferential surface of the balloon body and connected to the raised portions, fluid flow channels are defined between the covering membrane and the recessed portions, the fluid flow channels are isolated from the inflation lumen.

2. The balloon catheter according to claim 1, wherein there are a plurality of raised portions, which are evenly arranged circumferentially around the balloon body in the cross-section of the balloon body.

3. The balloon catheter according to claim 1 or 2, wherein the number of the raised portions is equal to the number of the recessed portions.

4. The balloon catheter according to claim 3, wherein the number of the raised portions ranges from two to six.

5. The balloon catheter according to claim 1 or 2, further comprising supports provided in at least some of the recessed portions, the supports extending along an axis of the balloon body, and the supports connected to both the recessed portions and the covering membrane.

6. The balloon catheter according to claim 5, wherein each of the supports has a dimension measured along a radial direction of the balloon body, which is equal to a difference between a radius of a circle defined by apices of all the raised portions and a radius of a circle defined by bottoms of all the recessed portions.

7. The balloon catheter according to claim 5, wherein each of the supports has a cross-section in the shape of a circular annulus, an outer diameter of the circular annulus being equal to a difference between a radius of a circle defined by apices of all the raised portions and a radius of a circle defined by bottoms of all the recessed portions.

8. The balloon catheter according to claim 1 or 2, wherein a circle defined by apices of all the raised portions has a diameter in the range of 2 mm to 5 mm and a circle defined by bottoms of all the recessed portions has a diameter in the range of 1 mm to 2.5 mm.

9. The balloon catheter according to claim 1 or 2, wherein an outer surface of the covering membrane is provided with a drug coating.

10. The balloon catheter according to claim 1 or 2, further comprising a catheter assembly defining an inflation medium channel, wherein the balloon body is disposed over an outer circumferential surface of the catheter assembly around a distal end thereof, and the inflation lumen of the balloon body communicates with the inflation medium channel.

11. The balloon catheter according to claim 10, wherein the catheter assembly comprises an outer tube and an inner tube, the inner tube partially inserted within the outer tube, a distal end of the inner tube being located external to a distal end of the outer tube, wherein the inflation medium channel is defined between the inner tube and the outer tube; the balloon body is disposed over an outer circumferential surface of the inner tube around a distal end thereof; and a proximal end of the balloon body is connected to the distal end of the outer tube.
